# EUROPEAN PATENT SPECIFICATION

(11) **EP 2 802 259 B1**
(45) Date of publication and mention of the grant of the patent: **04.11.2015**
(21) Application number: 13700100.4
(22) Date of filing: 09.01.2013
(51) Int. Cl.: G01N 27/327, A61B 5/15, A61B 5/151, A61B 5/157

(54) **TESTING MEMBER CARTRIDGE**
PRÜFELEMENTKARTUSCHE
CARTOUCHE D'ÉLÉMENTS D'ANALYSE

(30) Priority: 10.01.2012 EP 12150617
(43) Date of publication of application: 19.11.2014
(73) Proprietor: Sanofi-Aventis Deutschland GmbH, 65929 Frankfurt am Main (DE)
(72) Inventor: RICHTER, Frank, 65929 Frankfurt am Main (DE); MACARTHUR, Ross, Sandbach Cheshire CW11 1DZ (GB); WOLSELEY-HEXT, Elizabeth Verity, Stafford Staffordshire ST17 0TE (GB); COWAN, Joseph David, Stone Staffordshire ST15 8XG (GB); SMITH, Lee Thomas, Stafford Staffordshire ST16 1RL (GB); MILLS, David John, Stafford ST18 9HW (GB)
(74) Representative: McDougall, James
(86) International application number: PCT/EP2013/050310
(87) International publication number: WO 2013/104673

(56) References cited:
- WO-A2-02/078533
- WO-A2-2010/056869
- US-A1- 2002 057 993
- US-A1- 2004 178 216
- US-A1- 2006 157 362
- US-A1- 2009 270 765

## Description

### Field of the Invention

The present invention relates to a testing member cartridge and in particular to a testing member cartridge having contacts on an inner and outer surface of the cartridge.

### Background to the Invention

Diabetes sufferers may be provided with quantities of insulin, for instance by injection, sometimes a number of times daily. The quantity of insulin that is appropriate depends on the person's blood glucose level, so blood glucose level measurement can also occur a number of times daily.

Blood glucose level measurement typically is a multi stage process. The first is lancing, in which a lancet, or needle, is used to pierce the skin of a user, for example on the end or side of a finger. Once a suitable amount of blood has been produced, a sample is taken on a testing strip. A person may need to squeeze their finger in order to cause sufficient blood to be expelled. Sometimes lancing needs to be reperformed. The testing strip then is provided to a meter, typically an electronic meter, which analyses the sample, for example by determining a parameter (e.g. an electrochemical potential or voltage, resulting from a chemical reaction between the blood sample and an enzyme present in the testing strip, and provides a blood glucose measurement result. This measurement is then used to determine an amount of insulin to be consumed by the person.

Unpublished PCT patent applications numbered PCT/EP2011/061536, PCT/EP2011/061537, PCT/EP2011/061538, PCT/EP2011/061540 and PCT/EP2011/061542 and European applications numbered EP11182381.1, EP11182383.7 and EP11190679.8 relate to a new class of blood glucose measurement device. The device includes lancing and measuring features. In use, a user places a body part against an aperture in the device and the device first lances the body part then collects a blood sample, then processes the blood sample to measure a blood glucose level.

### Summary of the Invention

A first aspect of the invention provides a cartridge configured to be inserted into a meter, the cartridge comprising:
a housing having at least two electrical contacts disposed on an inner surface of a wall of the housing and at least two electrical contacts disposed on an outer surface of the wall of the housing, wherein a conductive path is provided between corresponding ones of the at least two electrical contacts on the inner and outer surfaces; and
at least one testing member supported within the housing, each testing member having at least two contact pads configured to engage with the at least two electrical contacts disposed on the inner surface of the housing wall when that testing member is moved into a measurement position, wherein the at least two electrical contacts disposed on the outer surface of the housing wall are configured to provide a conductive connection between the testing member and the meter.

The at least two contact pads of each testing member may be disposed on an edge of each testing member. The wall of the housing may be a side wall.

The housing may have three electrical contacts disposed on an inner surface of the wall of the housing and three electrical contacts disposed on an outer surface of the wall of the housing.

The contacts disposed on the inner surface may be brush contacts. Alternatively, the contacts disposed on the inner surface may be spring contacts.

A second aspect of the invention provides a cartridge according to the first aspect of the invention and a meter configured to retain the cartridge, the meter configured:
to detect whether the cartridge is correctly inserted into the meter; and
to permit movement of the testing members only when it is determined that the cartridge is correctly inserted.

The meter may further comprise at least two contacts configured to engage with the at least two electrical contacts disposed on the outer surface of the wall of the housing when the cartridge is correctly inserted so as to provide the conductive connection between the testing member and the meter.

The meter may further comprise a proximity sensor configured to enable the meter to detect the presence of the cartridge within the meter.

The meter may further comprise a mechanical switch configured to be engaged by the housing of the cartridge such as to enable the meter to detect the presence of the cartridge within the meter.

Each testing member may support a lancet which protrudes form an edge of each testing member.

### Brief Description of the Drawings

Embodiments of the invention will now be described, by way of example only, with reference to the accompanying drawings, in which:
Figure 1 is a perspective view of a blood glucose meter (BGM) according to aspects of the invention.
Figure 2 is a perspective view of the BGM of Figure 1 with a portion shown as transparent, so as to allow features inside a housing to be seen;
Figure 3 is the same as Figure 2 although a lid portion is shown as being removed;
Figure 4 is the same as Figure 3, although a cartridge is shown as partly removed;
Figure 5 illustrates components of one embodiment the BGM of Figure 1;
Figure 6 is a perspective view of components of the BGM of Figure 5 but with a hollow cylindrical housing part shown as transparent;
Figure 7 is a perspective view of a test disc member forming part of the BGM of Figures 1 and 5;
Figure 8 is an underneath perspective view of the test disc member of Figure 7;
Figures 9 to 12 illustrate the BGM of Figures 5 to 7 at different stages of a blood collection sample process;
Figure 13 is an alternative embodiment of a test disc member;
Figure 14 is a schematic view of the outside of the cartridge;
Figure 15 shows a cross-section through the cartridge; and
Figure 16 is a flowchart illustrating operation of the first embodiment of the BGM of Figure 1.

### Description of Embodiments of the Invention

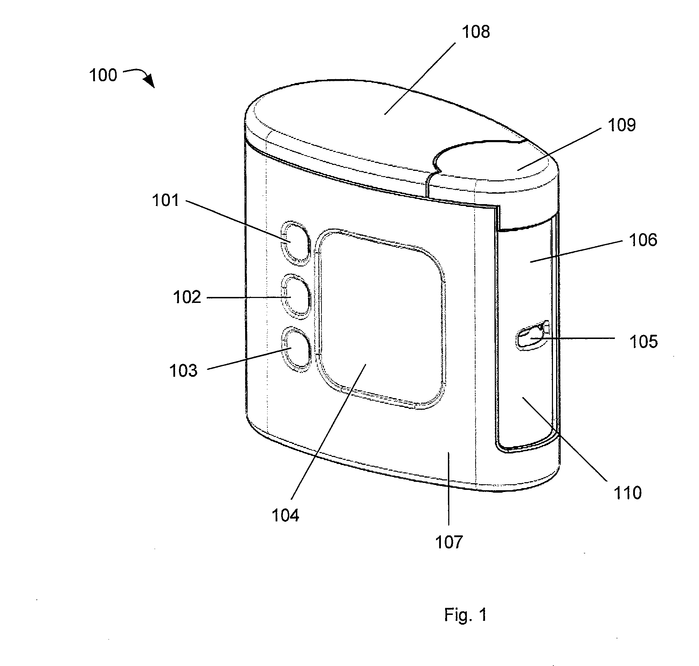

A blood glucose meter (BGM) 100 is shown in Figure 1. The BGM 100 is shown in a perspective view. The BGM 100 has a generally flat base, that is not visible in the figure. The BGM 100 is approximately as tall as it is long, and its width is approximately one-third of its height

On one side face of the BGM are provided first, second and third inputs 101, 102, 103. These may take the form of push-switches or touch sensitive transducers, for instance. Also provided on the side of the BGM next to the input devices 101 to 103 is a display 104. This may take any suitable form, for instance a liquid crystal display (LCD), e-ink etc. In use, a user may control the BGM 100 using the input devices 101 to 103 and may be provided with information by the BGM through the display 104.

Located at a front face of the BGM 100 is an aperture 105. The aperture 105 is located at approximately half of the height of the BGM. The aperture 105 is configured such as to be able to receive a part of a user's body, for the purpose of extracting a blood sample therefrom. For instance, the aperture 105 may be dimensioned so as to receive an end or a side part of a finger or thumb, or may be dimensioned so as to receive a side of a user's hand or a pinch of skin from a user's arm. The aperture may be rectangular in shape. Its edges may be bevelled, so as to guide a user's digit into a specific location.

The aperture 105 is provided in the side of a cartridge 106. The cartridge has a generally cylindrical form, and is arranged vertically in the BGM 100.

In particular, the BGM includes a first housing part 107. The first housing part 107 forms the base, left and right side face and the rear face of the BGM 100. On the front face of the BGM 100, the first housing part 107 also comprises the lowermost part of the side face. A fixed lid part 108 is attached to the first housing part 107. The fixed lid part 108 comprises most of the top surface of the BGM 100. A removable lid part 109 comprises the remaining part of the top surface of the BGM 100. The removable lid part is disposed above the cartridge 106 at the front face of the BGM 100.

The first housing part 107 is configured such as to provide an elongate aperture 110 at the front face of the BGM 100. The elongate aperture 110 may extend for most of the height of the front face of the BGM 100. The elongate aperture 110 is defined at the uppermost part by the removable lid part 109 and is defined by the first housing part 107 at the right, left and bottom. The BGM 100 is arranged such that the cartridge 106 occupies the whole of the area of the elongate aperture 110. A slidable or pivotable door in the housing part 107 of the BGM 100 may cover all or a part of the elongate aperture 110 when the BGM is not in use. The door may cover at least the aperture 105, such as to prevent the ingress of dirt and other potential contaminants into the aperture 105

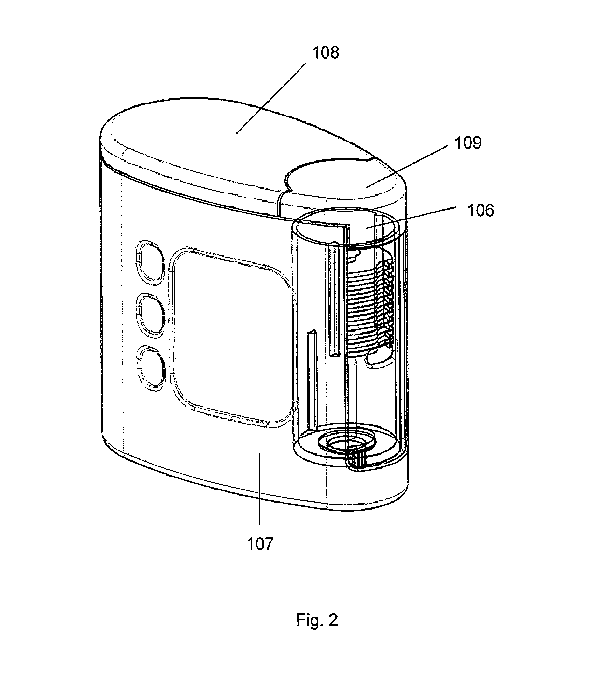

The cartridge 106 is more clearly visible in Figure 2. Figure 2 shows the same view as Figure 1, although the removable lid part 109 and the first housing part 107 are shown in wire frame. As can be seen from Figure 2, the cartridge 106 has a generally cylindrical form, and is arranged vertically. The diameter of the cartridge 106 is greater than the width of the aperture 110 by a factor for instance of between 5 and 50%. The cartridge 106 has a length that is between 3 or 4 times its diameter.

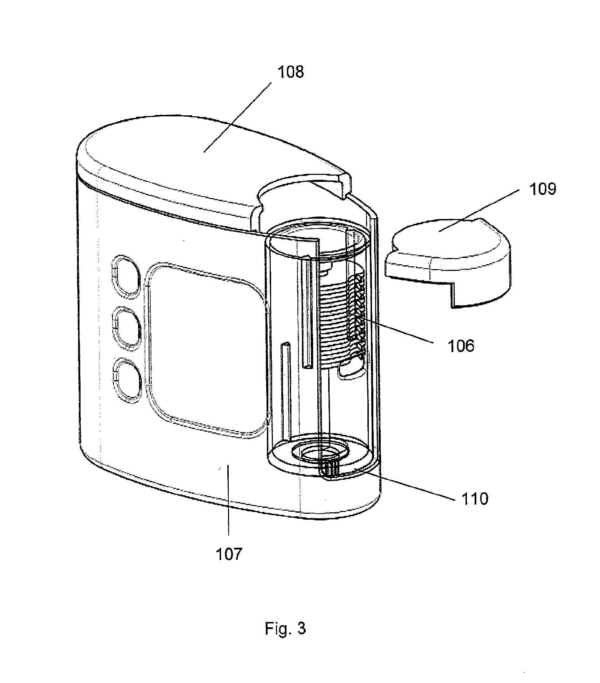

In Figure 3, the removable lid part 109 is shown as having been removed from the BGM 100. The first housing part 107, the fixed lid part 108 and the removable lid part 109 are configured such that when the removable lid part is in place on the BGM the cartridge 106 is retained by mechanical interaction between the three components but is removable by a user. The exact way in which the removable lid part 109 is released from the BGM 100 is not critical and is not described in detail here.

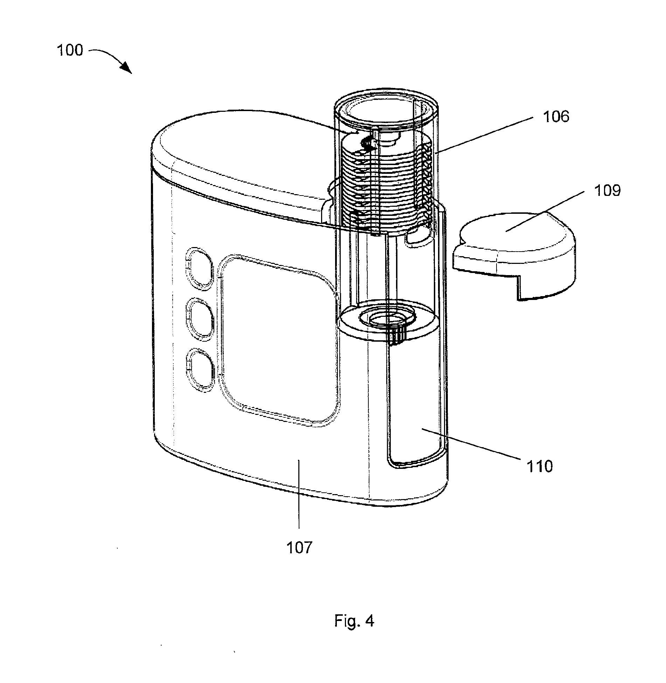

The removable lid part 109 is configured such that when removed from the BGM 100 the cartridge 106 is able to be extracted from the BGM by moving it vertically along its axis. In Figure 4, the cartridge 106 is shown as being partly removed from the BGM 100. When fully removed, the elongate aperture 110 reveals a cavity in the BGM 100. A replacement cartridge can then be introduced into the BGM 100 in the opposite manner to which the old cartridge 106 was removed. Once located at the bottom of the cavity in the BGM, the new cartridge 106 is partly surrounded by the first housing part 107. Once the removable lid part 109 has been replaced, to the position shown in Figure 1, the cartridge 106 is retained in place by the action of the first housing part 107 and the removable lid part 109. The aperture 105 in the cartridge 106 is presented at the front face of the BGM 100, in the same way as shown in Figure 1. The cartridge 106 and the cavity which receives the cartridge may have a keying feature, such as a protrusion and a groove, a non circular diameter, or the like. Thus, when the cartridge 106 is fully inserted, the aperture 105 is in a fixed position to the elongate aperture 110, for example in a centred position as shown in Fig. 1.

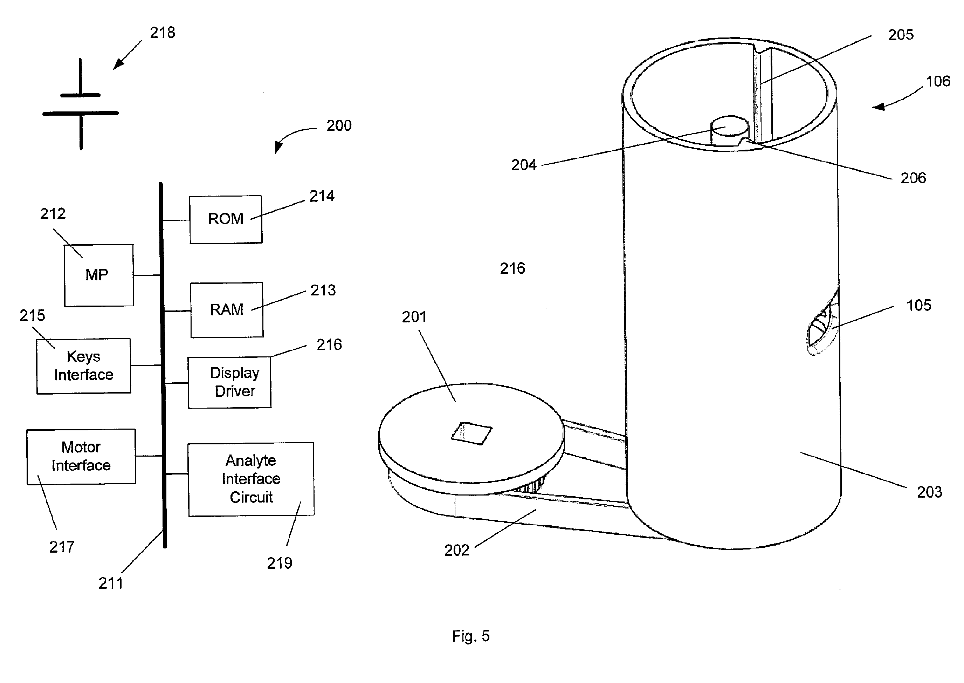

Figure 5 shows a subsystem 200 of the blood glucose meter 100. The subsystem 200 includes the cartridge 106, a drive wheel 201 and a drive belt 202.

In Figure 5, the cartridge shown as having a hollow cylindrical housing part 203, which constitutes part of a housing. The aperture 105 is formed in the hollow cylindrical housing part 203. Coaxial with the hollow cylindrical part 203 is an elongate shaft 204, only the top part of which is illustrated in Figure 5. The length of the shaft 204 is such that its uppermost end is slightly below the uppermost end of the hollow cylindrical housing part 203. As will be described below, the shaft 204 is mechanically coupled with the drive belt 202 so as to be rotatable by rotation of the drive wheel 201.

Formed with the inner surface of the hollow cylindrical housing part 203 are first and second guide members 205, 206. In Figure 5, it can be seen that the first and second guide members 205, 206 have a generally triangular cross section. One side of the triangular cross section of the first and second guide members 205, 206 is integral with the inner surface of the hollow cylindrical housing part 203, with a point of the triangular cross section extending towards the centre of the cartridge 106. A part of the length of the first guide member 205 is visible in Figure 5, but only the uppermost surface of the second guide member 206 is visible in that figure.

Figure 5 also shows some electronic components that form parts of the blood glucose meter 100. These components are provided within the housing 107 but do not form part of the cartridge 106.

A bus 211 is arranged to connect a number of components including a microprocessor 212, random access memory (RAM) 213, read-only memory (ROM) 214, a keys interface 215, a display driver 216, an analyte interface circuit 219 and a motor interface 217. All of these components are powered by a battery 218, which may take any suitable form.

Stored in the ROM 214 is software and firmware that governs operation of the blood glucose meter 100. The software/firmware is executed by the microprocessor 212 using the RAM 213. The software/firmware stored in the ROM 214 is operable to operate the blood glucose meter 100 such as to allow control by a user through the keys or input devices 101 to 103, as detected by the keys interface 215. A blood glucose measurement and other information is provided on the display 104 at suitable times by operation of the software/firmware and the microprocessor 212 through the display driver 216.

The motor interface 217 allows the microprocessor 212, according to the software/firmware stored in the ROM 214, to control the motor that is coupled to the drive wheel 201, and any other motors that are included in the blood glucose meter 100 (as will be described below).

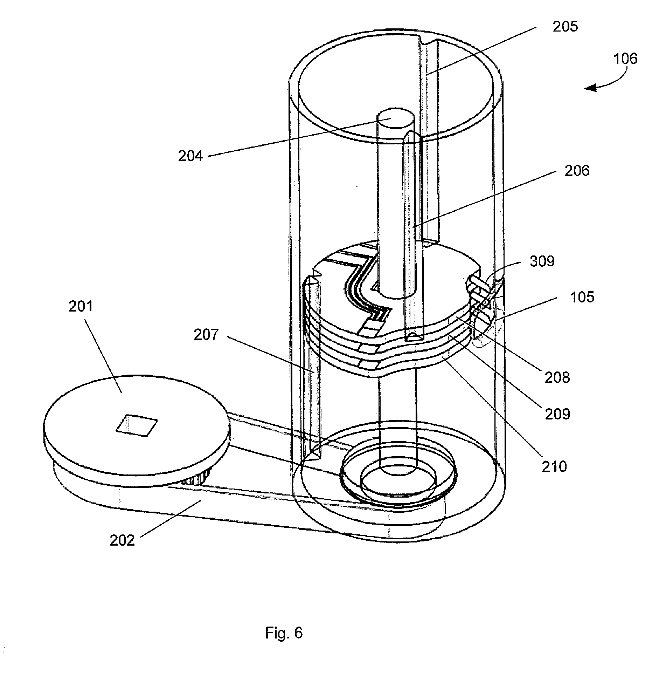

The analyte interface circuit 219 is operable to provide electrical signals with certain voltages to the electrical contact terminals 401, and thus the contact pads 318 (described in detail with respect to Figures 6 to 13) and thus the analyte measuring part 316, and to measure parameters of signals such as to allow the microprocessor 212 to measure a blood glucose level of a blood sample.

Figure 6 is the same as Figure 5 except that the hollow cylindrical housing part 203 is shown in wire frame, so as to reveal components internal to it, and in that the electronic components are omitted. In Figure 6, a third guide member 207 is visible. As can be seen from this figure, the first and second guide members 205, 206 are located only in the uppermost half of the length of the cartridge 106, and the third guide member 207 is located only in the lowermost half of the cartridge 106. The first, second and third guide members 205 to 207 are distributed around the circumference of the hollow cylindrical housing part 203. In particular, the first and second guide members 205, 206 are located at approximately 100 to 160 degrees from one another. The third guide member 207 is located approximately 60 to 130 degrees from each of the first and second guide members 205, 206.

Mounted on the shaft 204 are a plurality of members, three of which are shown in Figure 6 as 208, 209 and 210 respectively. The members 208 to 210 will hereafter be referred to as test disc members. Each of the test disc members 208 to 210 is substantially the same.

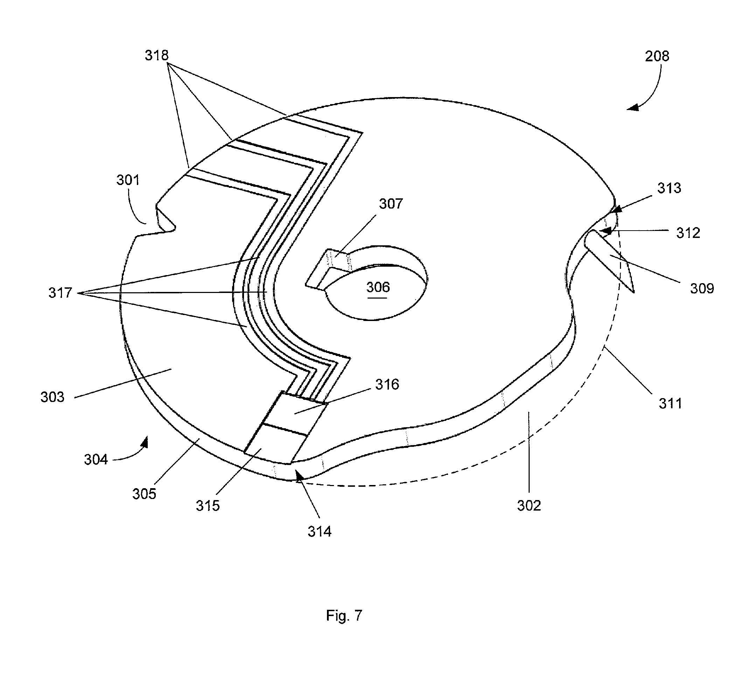

One test disc member 208 is shown in some detail in Figure 7. The test disc member 208 has a generally circular shape, although on one side a notch 301 is formed and on another side a cutaway portion 302 is provided. The cutaway portion constitutes a milking portion, and will be described in more detail below.

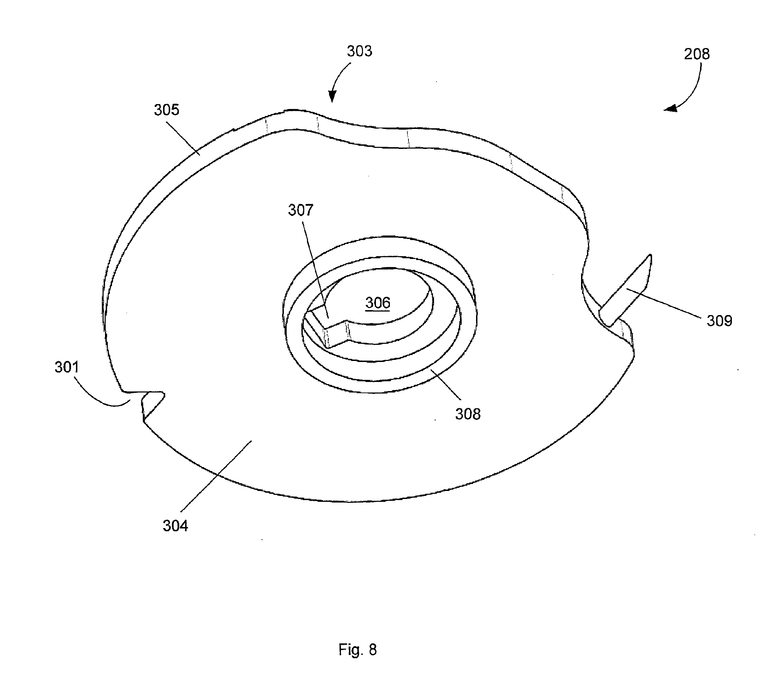

The test disc member 208 includes an uppermost surface 303, a lowermost surface 304, which is shown in Figure 8, and a disc edge 305. The diameter of the test disc member 208 is between 15 and 25 millimetres, for instance 20 millimetres. The thickness of the disc, which is equal to the height of the disc edge 305, is between 0.5 millimetres and 1 millimetre. Figure 8 shows the test disc member 208 from the underside. As such, the lower surface 304 is visible and the upper surface 303 is not visible. The test disc member 208 will now be described with reference to Figures 7 and 8.

A hole 306 is formed at the centre of the test disc member 208. The hole 306 comprises two main parts. A circular part is centred on the test disc member 208 and has a diameter equal to or slightly larger than the external diameter of the shaft 204. A drive notch 307 abuts the circular part of the hole 306 and includes edges that are able to be engaged by a drive dog.

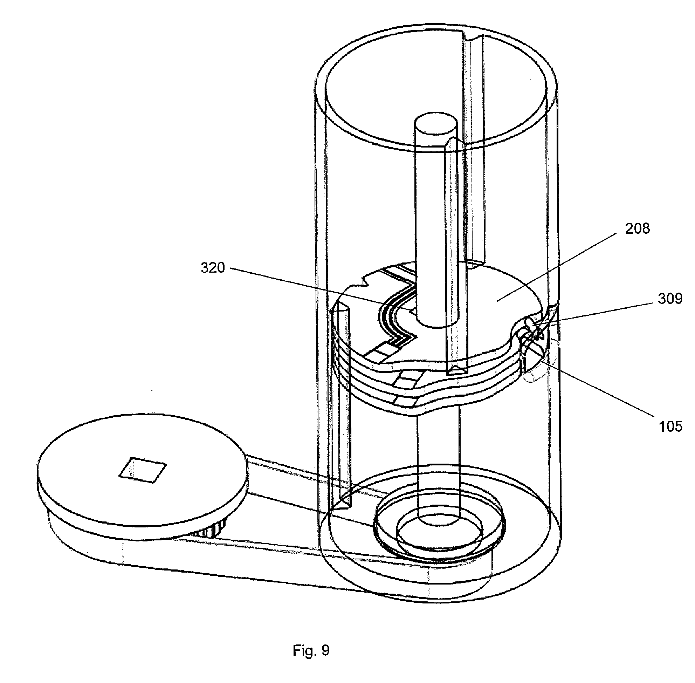

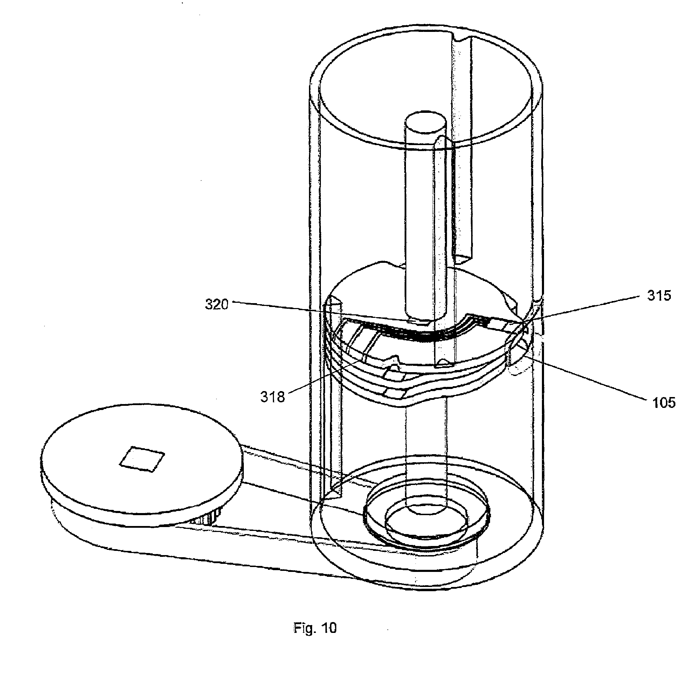

A drive dog 320(visible in part in Figure 9 and more fully in Figure 10) is formed on the shaft 204. The drive dog 320 is engaged with the drive notch 307 in the hole 306 of the test disc member 208. This engagement allows rotation of the shaft 204 to result in rotation of the test disc member 208.

On the underside of the test disc member 208 is provided a spacer member 308. The spacer member 308 comprises a slice of a hollow cylinder. The cylinder is centred on the centre of the test disc member 208. The inner diameter of the spacer member 308 is selected such that the hole 306 does not overlap with the spacer member 308. The outer diameter of the spacer member 308 is only slightly greater than the inner diameter, so the spacer member 308 has little thickness. The height of the spacer member 308 is between 0.5 and 1 millimetre. When plural test disc members are stacked together, the spacer member 308 provides separation between the upper surface 303 of one test disc member and the lower surface 304 of the test disc member that is directly above it. The separation is determined by the height of the spacer member 308.

Referring again to Figure 7, a lancet 309 is shown protruding from the disc edge 305. The lancet 309 is provided in the cutaway portion 302. A first end of the lancet 309 is embedded within the material of the test disc member 208, and a second end is provided with a sharp point and extends outwardly. The lancet 309 extends at an angle between 30 and 60 degrees from a radius line of the test disc member 208 at the position where the end of the lancet 309 is embedded in the test disc member. The second end of the lancet 309 is located at or just outside a circumference 311 of the test disc member 208. The circumference 311 is shown as a dotted line in Figure 7 because it is virtual, instead of tangible. The lancet 309 extends from the disc edge 305 at a first position 312 on the disc edge. The first position 312 is close to a second position 313 at which the cutaway portion 302 starts. The cutaway portion 302 ends at a third position 314. Between the second and third positions 313, 314 opposite to the cutaway portion 302, the disc edge 305 generally takes the form of a circle, although the notch 301 interrupts that circle.

Located next to the third position 314 is a blood collection part 315. This may take any suitable form. For instance, it may comprise a laminated material. The blood collection part 315 has the function of drawing blood that is in contact with the disc edge 305 at the third position into the test disc member 208 to an blood analyte measuring part 316, that adjoins the blood collection part 315, for example a part containing an enzyme for blood glucose measuring, or the like. Blood may be drawn through capillary action. The analyte measuring part 316 includes an enzyme that reacts chemically with blood in such a way that blood glucose level can be measured. The analyte measuring part 316 is connected to first to third contact pads 318 by first to third conductive tracks 317. The contact pads 318 are formed on the edge 305 of the test disc member 208. The conductive tracks 317 are formed on the upper surface 303 of the test disc member 208. The analyte measuring part 316 also is formed on the upper surface 303 of the test disc member 208. Some or all of the conductive tracks 317, the contact pads 318 and the analyte measuring part 316 may be printed onto the test disc surface. In some alternative embodiments, only two contact pads 318 and two conductive tracks 317 are provided.

As will be described in detail below, in use a part of a user is firstly pierced by the lancet 309, the part is then milked by the disc edge 305 at the cutaway portion 302, and blood then is provided to the analyte measuring part 316 through the blood collecting part 315. A measuring circuit connected to the analyte measuring part 316 by way of the conductive tracks 317 and the contact pads 318 then is able to determine a blood glucose level of the user. The level then is displayed on the display 104.

Operation will now be described with reference to the figures.

As shown in Figure 6, the test disc members 208 to 210 commence at the same orientation. Here, the first test disc member 208 is uppermost. The third guide member 207 is located in the notch 301 of the lowermost test disc members 209, 210. The notch 301 of the first test disc member 208 is aligned with the third guide member 207, but is not constrained thereby. The upper surface 303 of the uppermost test disc member 208 is in contact with a lowermost surface of the first guide member 205. The lowermost surface of the second guide member 206 is at the same level as the lowermost end of the first guide member 205. However, the second guide member 206 coincides with part of the cutaway portion 302 of the first test disc member 208 at the orientation of the test disc member 208 shown in Figure 6. As such, there is no contact between the second guide member 206 and the first test disc member 208 when the first test disc member is in this position. The test disc members 208 to 210 are biased in an upwards direction by bias means (not shown), which may be a spring. However, the test disc members 200 to 210 are prevented from moving upwards within the cartridge 106 by virtue of the contact between the upper surface 303 of the first test member 208 and the lowermost end of the first guide member 205.

At the position shown in Figure 6, the distal end of the lancet 309 is not co-located with the aperture 105. As such, the lancet 309 is in this position not operational. Put another way, the lancet 309 at this position is shielded by the hollow cylindrical part 203, which constitutes part of the housing.

From the position shown in Figure 6, the shaft 204 is caused to rotate in a clockwise direction by action of the drive wheel 201 and drive belt 202. The drive dog 320 is engaged with the drive notch 307 in the hole 306 of the test disc member 308, and so allows rotation of the shaft 204 to result in rotation of the test disc member 308. Rotation brings the lancet 309 in front of the aperture 105. As such, a skin-covered part of a user (hereafter the part will be referred to as a user's digit, for the sake of convenience) is lanced by the lancet 309. This produces a puncture in the skin of the digit, through which blood can escape. Figure 9 shows the first test disc member 208 rotated to the position where the lancet 309 is operable to lance the user's digit. The shaft 204 is caused to rotate only by a predetermined amount, the maximum extent of travel of the lancet 309 is controlled. The penetration of the lancet 309 in the user's digit depends on a number of factors, as will be appreciated by the person skilled in the art. The amount of rotation, and thus the depth of penetration, may be definable by a user.

Subsequently, the shaft 204 is controlled to rotate in an anticlockwise direction. This causes the lancet 309 to be removed from the user's digit, and for the disc edge 305 at the cutaway portion 302 to rub the user's digit as the test disc member 208 rotates.

At a point in the rotation of the test disc member 208, the lowermost part of the second guide member 206 ceases to coincide with the cutaway portion 302 and so is able to exert a reaction force on the upper surface 303 of the test disc member 208. A short time thereafter, the lowermost part of the first guide member 205 becomes coincident with the cutaway portion 302, and ceases to contact the upper surface 303 of the test disc member 208. At this point, it is the second guide member 206 that prevents the first test disc member 208 moving upwards within the cartridge 206.

The test disc member 208 continues to rotate until the blood collection part 315 is aligned with the aperture 105. Here, rotation ceases. At this location, blood that has been caused to be expelled from the user's digit by the lancet 309 and by action of the disc edge 305 on the user's digit is caused to be drawn to the analyte measuring part 316 by capillary action. The blood and the enzyme then react.

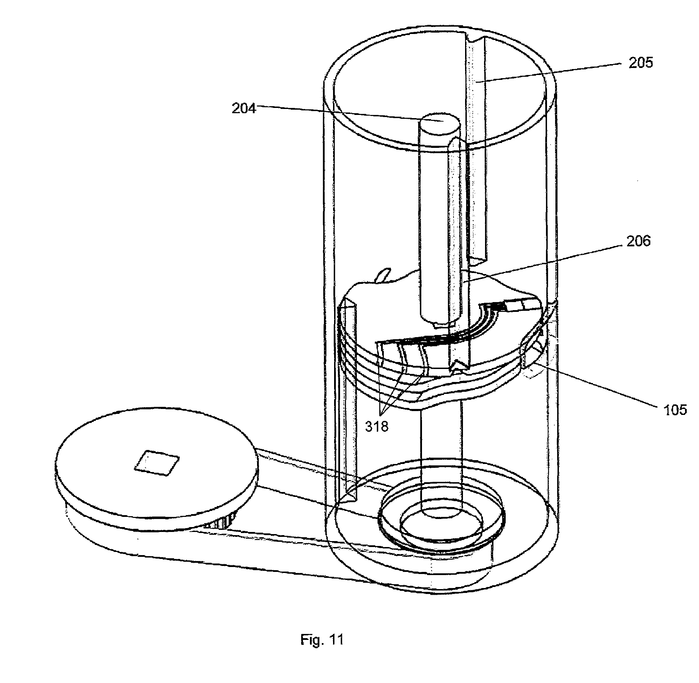

At a suitable time, the shaft 204 is caused to be rotated further in an anticlockwise direction. Here, the test disc member 208 is caused to be rotated from the position shown in Figure 10, in which the blood collection part 315 is coincident with the aperture 105, to the position shown in Figure 11. Here, the notch 301 is aligned with the second guide member 206. Because at this location the first guide member 205 is coincident with the cutaway portion 302 of the test disc member 208, neither of the first or second guide members 205, 206 prevents upwards movement of the first test disc member 208. As such, the first to third disc members 208 to 210 are moved upwards by virtue of the bias means (not shown).

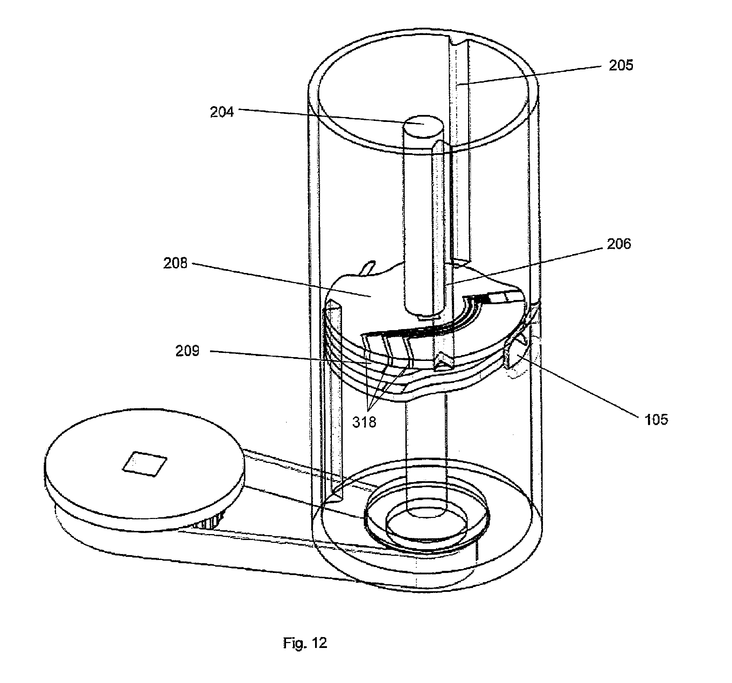

When the first test disc member 208 moves upwards, between Figures 11 and 12, the drive dog 320 ceases to cooperate with the drive notch 307 of the hole 306 of the first test disc member 208. Before the first test disc member 208 reaches the position shown in Figure 12, a lower surface of the drive dog 320 contacts the upper surface 303 of the second test disc member 209. This prevents further upward movement of the second test disc member 209, and thus prevents further movement of the test disc member 210. At this position, the shaft 204 is caused to be rotated by the drive wheel 201 and the drive belt 202 such that the drive dog 320 is coincident with the drive notch 307 of the second test disc member 209. At this location, the second disc member 209 is able to move upwards on the shaft 204, thereby engaging the drive dog 320 with the drive notch 307 of the second test disc member 209. After the second test disc member 209 has moved upward by a distance equal to the height of the spacer member 308, further upwards movement of the second test disc member 209 is prevented by contact between the first guide member 205 and the upper surface 303 of the second test disc member 209. At this point, which is shown in Figure 12, the second guide member 206 is located within the notch 301 of the first test disc member 208. This prevents further rotation of the first test disc member 208 within the cartridge 106.

By virtue of movement up the cartridge 106 of the first to third test disc members 208 to 210, the third guide member 207 ceases to be within the notch 301 of the second test disc member 209. At this stage, the third guide member 207 does not prevent rotational movement of the second disc member 209.

At the position shown in Figure 12, the second test disc member 209 is in exactly the same position as was the first test disc member 208 at the position shown in Figure 6. Furthermore, the shaft 204, and thus the drive dog 320, has the same orientation. As such, the second test disc member 209 is able to be used to elicit a blood sample from a user and test the glucose level thereof in the same way as was the first test disc member 208.

By providing a stack of test disc members 208 to 210 within the cartridge 106 and by providing a suitable physical arrangement, a cartridge 106 can be used for multiple tests. When the cartridge 106 is new, the test disc members 208 to 210 are located in the bottom half of the cartridge 106, with the uppermost test disc member being aligned with the aperture 105. As test disc members are used, the stack of test disc members moves upwards in the cartridge. When the last test disc member is used, the cartridge can be said to be spent. At this stage, all of the test disc members are located in the uppermost portion of the cartridge 106.

It will be appreciated that the number of test disc members 208 to 210 that can be accommodated within the cartridge 106, and thus the number of tests that can be provided by a cartridge 106, is a factor of the height of the cartridge 106, and the separation between corresponding parts (e.g. the upper surfaces) of adjacent test disc members 208 to 210. A taller cartridge and/or a reduced separation of test disc members increases the number of tests that can be performed using a single cartridge 106.

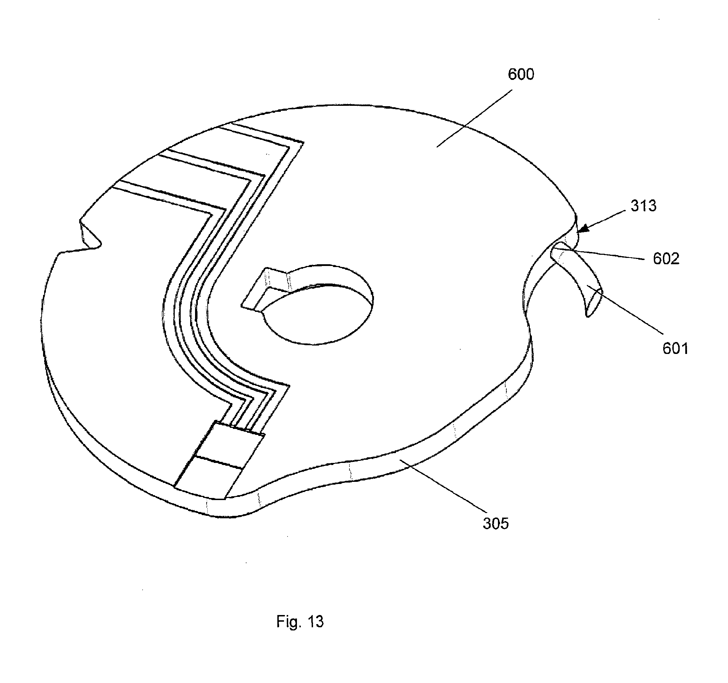

An alternative form of test disc member 600 is shown in Figure 13. Reference numerals are retained from above-described embodiments for like elements.

The test disc member 600 differs from the test disc member 208 shown in Figure 7 primarily by use of a curved lancet 601. The curved lancet 601 protrudes from the disc edge 305 at a position 602 that is relatively close to a second position 313 at which the cutaway portion 302 commences.

At the part of the curved lancet 601 that is adjacent the disc edge 305, the longitudinal axis of the curved lancet 601 is at an angle X with respect to a straight line drawn between the junction between the curved lancet 601 and the disc edge 305 and the centre of the shaft 204. The curve of the curved lancet 601 is such that the longitudinal axis of the curved lancet at the end distant from the disc edge 305 is at an angle greater than the angle X with respect to the line drawn between the junction between the curved lancet 601 and the disc edge 305 and the centre of the shaft 204. The effect is that the curved lancet 601 is more aligned with the circumference of the test disc member 600 at its distal end than it is at the end that adjoins the disc edge 305. This has the positive effect that when the lancet penetrates a user's digit, or other body part, due to rotation of the test disc member 600, the path taken by the lancet as it penetrates the user's digit more closely matches the shape and orientation of the lancet than is experienced in a corresponding arrangement with a straight lancet.

This effect is enhanced with the lancet 601 since the cylindrical form of the lancet 601 is terminated at the distal end by an oblique cut. In particular, the distal end of the curved lancet 601 resembles a cylinder that has been cut at an angle that is not perpendicular to the longitudinal axis of the cylinder. As such, the end face of the curved lancet 601 has the shape of an ellipse. The ellipse has a semi-major axis and a semi-minor axis and the point that is at the end of the semi-major axis that is furthest from the disc edge 305 forms a point. The cut is made through the lancet 601 such that the point is formed extending in a direction that is substantially circumferential with respect to the test disc member 600.

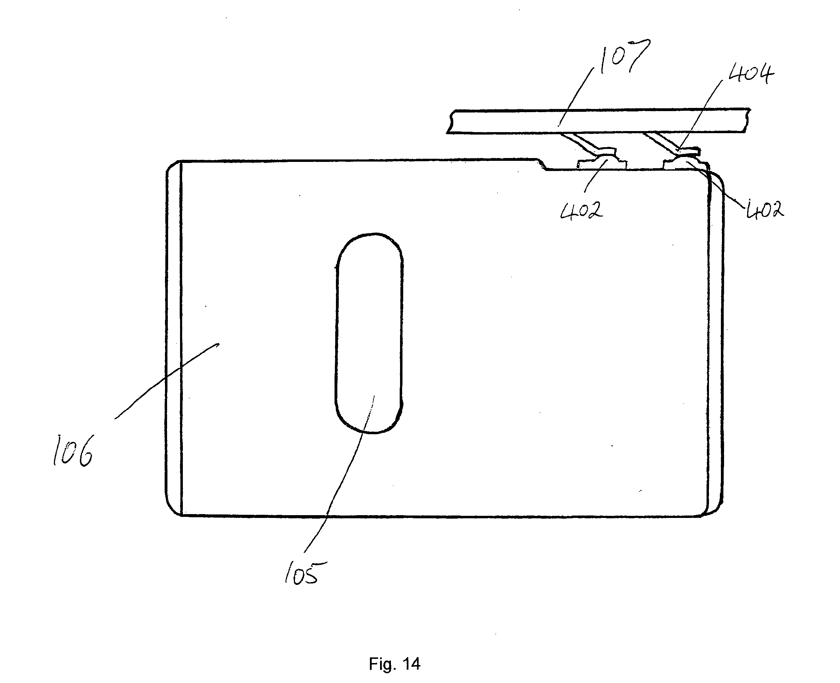

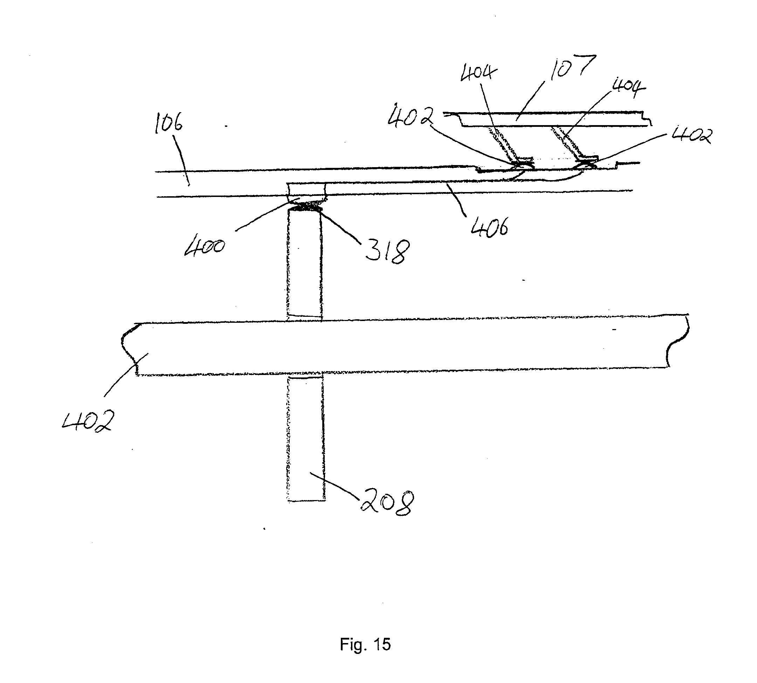

Reference will now be made to Figures 14 and 15, which illustrate connection of the analyte measuring part 316 to measurement circuitry (not shown).

Figure 14 shows a schematic view of the outside of the cartridge 106 and the aperture 105 in the cartridge 106. The cartridge 106 has a generally cylindrical shape as previously described. A pair of contacts 402 (also referred to herein as outer surface contacts 402) are located on the outer surface of the side wall of the cartridge. These contacts 402 may be located close to the bottom of the side wall as shown in Figure 14, or alternatively may be located at any vertical position on the side wall of the cartridge 106.

A portion of the housing 107 of the BGM 100 is shown in Figure 14. This portion of the housing 107 supports a pair of contacts 404 (also referred to herein as housing contacts 404). When the cartridge 106 is inserted into the BGM 100, the outer surface contacts 402 engage with the housing contacts 404. The housing contacts 404 may be sprung contacts such that they are biased against the outer surface contacts 402. This ensures a good electrical connection between the two pairs of contacts 402, 404. The cartridge 106 and the housing 107 may also have more contacts, for example, three, four or more contacts.

Figure 15 is similar to Figure 14, but shows a cross-section through the cartridge 106. The spindle 402 is disposed centrally within the cartridge. A first test disc 208 is rotatably mounted on the spindle. Only one test disc 208 is shown in Figure 15 for clarity. As previously stated, contact pads 318 are located on the disc edge 305. Conductive tracts 317 connect these pads 318 to the analyte measuring part 316.

The internal surface of the cartridge 106 supports a number of contacts 400 (also referred to herein as inner surface contacts 400). The inner surface contacts 400 are embedded in the cartridge wall and protrude from the inner surface of the cartridge wall. The number of inner surface contacts 400 supported by the cartridge housing corresponds to the number of contact pads 318 on the test disc member 208. For example, there may be two or three inner surface contacts 400 which engage with respect ones of the contact pads 318. The inner surface contacts 400 may be brush contacts. This allows the contact pads 318 to move whilst in contact with the inner surface contacts 400. Alternatively, the inner surface contacts 400 may be spring type contacts. These also allow the contact pads 318 to move whilst in contact with the inner surface contacts 400.

The inner surface contacts 400 are electrically connected to the outer surface contacts 402 by a conductive pathway 406 with passes through the side wall of the cartridge 106. This pathway 406 may be a wire or any other suitable conductive track.

In use, a user inserts a cartridge 106 into the BGM 100. A protrusion (not shown) on the outer surface of the cartridge 106 may engage with a groove (not shown) in the BGM 100 to ensure correct orientation of the cartridge 106 within the BGM 100. The outer surface contacts 402 on the cartridge 106 engage with the housing contacts 404 which protrude from the inner surface of the housing 107.

The housing 107 of the BGM 100 may additionally support a proximity sensor (not shown). This sensor may be connected to and controlled by the microprocessor 212. The proximity sensor may be supported by the inner surface of the BGM housing 107 and may be directed inwardly, towards the cartridge cavity. The microprocessor 212 may receive signals from the proximity sensor in order to determine whether a cartridge 106 is present within the BGM 100. The housing 107 of the BGM 100 may alternatively or additionally support a mechanical switch (not shown). The mechanical switch may be an electromechanical switch and may be connected to the microprocessor 212. The mechanical switch may have an arm which protrudes into the cartridge cavity such that it is contacted by the base of the cartridge 106 as the cartridge is inserted. This contact may activate the switch causing a signal to be sent to the microprocessor 212, from which the microprocessor 212 can determine that a cartridge has been inserted.

At some later point, the user performs a blood collection and analysis operation. When this operation reaches the point shown in Figure 10, the blood collection portion 315 is aligned with the aperture 105 and in contact with the user's digit. At this position the contact pads 318 become aligned with (and are in contact with) the inner surface contacts 400 of the cartridge. Thus a complete electrical circuit exists which connects the blood analyte part 316 to the measurement circuitry within the BGM 100, via the conductive tracks 317, contact pads 318, inner surface contacts 400, conductive pathway 406, outer surface contacts 402 and housing contacts 404. This complete circuit exists only while the test disc member 208 is in this rotational position.

Having a conductive pathway 406 which passes through the side wall of the cartridge 106 allows an electrical connection to be made between the test disc member 208 and the BGM 100 without the need for any additional moving parts or any additional apertures in the cartridge 106. Thus a sterile and low humidity environment is more easily maintained within the cartridge 106.

Instead of being supported by the housing 107, the contacts 404 may be supported by a member which protrudes internally from the housing 107.

Additional contacts may be provided on the cartridge to allow identification of the cartridge, or to read out information related to the cartridge. For example, a certain resistance or impedance which can be measured between additional contacts may be used to identify the cartridge. Alternatively, the additional contacts may be connected to a memory unit that stores information related to the cartridge, for example a lot number, an accuracy range of the test members in the cartridge, or a calibration value that may be applied to measurement results from the test members in order to make up for fabrication tolerances. The information may be stored into the memory unit during or after production of the cartridge.

The configuration of the test disc members 208 to 210, 600 is such that operation results in milking of the puncture in the user's digit caused by the lancet 309. In particular, the aperture 105 is configured such as to allow an amount of the flesh making up the end of the user's digit to be present within the internal volume of the cylindrical part 203 when the user presses the digit up against the aperture 105. When the user applies force into the aperture 105 with the digit, the digit distorts and a bulbous part is provided within the internal diameter of the hollow cylindrical housing part 203. The size of the bulbous part, and in particular the height of the bulbous part, depends on a number of factors, including the physical characteristics of the user's digit and the amount of force that the user applies, as well as the configuration of the aperture 105.

The aperture 105 is dimensioned such that in normal use (i.e. with a typical user applying a typical amount of force) a bulbous part of the user's digit extends into the internal volume of the hollow cylindrical housing part 203 to a depth of approximately 1 millimetre. The test disc members 208 to 210, 600 are configured to have a cutaway portion 302 that is shaped such that when the lancet 309 is at a position at which it can lance the user's digit, the disc edge 305 is not in contact with the user's digit (i.e. the separation between the disc edge 305 and the aperture 105 is greater than 1 mm). This part of the cutaway portion 302 can be termed a first milking portion. At this position, the pressure exerted by the user results in the fluid pressure within the bulbous part of their digit being slightly greater than normal pressure. The increased pressure results from the force the user applies to their digit. This pressure encourages bleeding of the puncture that is caused by the lancet 309. Advantageously, the arrangement of the relevant features is such that the lancet 309 penetrates the user's digit to a depth of between 0.4 and 0.7 millimetres.

As the test disc member 208 to 210, 600 then rotates anticlockwise, the lancet 309 is removed from the user's digit. A short time thereafter, the end of the bulbous part of the user's digit comes into contact with the disc edge 305 at a position approximately one-third to two-fifths of the way along the cut out portion 203. This part can be termed the second milking portion. The test disc member 208 to 210, 600 has a substantially constant radius for the second milking portion, which extends to a position approximately two-thirds or four-fifths of the way along the cutaway portion 302. For the time at which the second milking portion is coincident with the bulbous part of the user's digit as the test disc member 208 to 210 rotates, the internal pressure of the bulbous part of the user's digit is increased compared to the time at which the user's digit was in contact with the lancet 309. Furthermore, as the disc edge 305 moves into contact with and over the bulbous part of the digit, blood under the skin is caused to be pushed towards the puncture caused by the lancet.

Between the second milking part and the location of the blood collection part 315, the radius of the test disc member 208 to 210, 600 is reduced, or put another way has a lower value. This portion can be termed a third milking portion. As such, after the second milking portion and before the user's digit contacts the blood collection part 315, the pressure applied to the bulbous part of the user's digit by the disc edge 305 is reduced compared to the pressure applied at the second milking portion. Advantageously, the radius of the test disc member 208 to 210, 600 at the third milking portion is selected such that the bulbous part of the user's digit does not contact the disc edge 305 (i.e. the separation between the disc edge 305 and the aperture 105 is greater than 1 mm). Whilst the third milking portion is coincident with the user's digit as the test disc member 208 to 210, 600 rotates, blood is free to exit the puncture made by the lancet 309. As the test disc member 208 to 210, 600 continues to rotate, the disc edge 305 again contacts the bulbous part of the user's digit at a location just before the blood collection part 315. This again increases the internal pressure within the bulbous part of the user's digit. This encourages the movement of blood towards the analyte measuring part 316. The separation between the disc edge 305 at the location of the blood collection part 315 and the aperture 105 is approximately 0.5 mm.

The configuration of the test disc members 208 to 210, 600 thus encourages milking of a sample of blood from the user's digit. The sequence is as follows: Firstly, lancing by the lancet 309 with a relatively low pressure (caused by no contact with the disc edge 305 and the user's digit), followed by a period for which relatively low amount of pressure, as well as a rubbing movement, is provided by the second milking portion to the user's digit, followed by a period for which little or no pressure is provided by the disc edge 305 against the user's digit, followed by a relatively high pressure provided by the disc edge 305 against the user's digit just before and at the blood collection part 315.

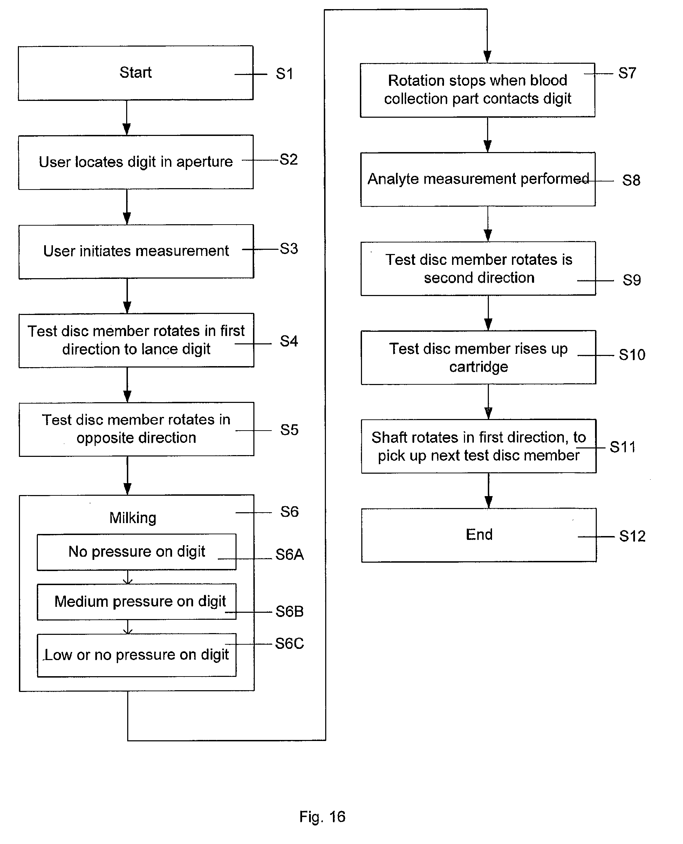

Operation of the blood glucose meter 100 will now be described with reference to the flowchart of Figure 16. Operation starts at step S1. At step S2, the user locates their digit in the aperture 105. As mentioned above, the user forces their digit into the aperture 105 with a pressure or force that is suitable to allow lancing and blood collection. At step S3, the user initiates blood glucose measurement. This involves the user pressing one of the inputs 101 to 103. This is detected by the microprocessor 212 by way of the keys interface 215. The software/firmware stored in the ROM 214 uses the key input to call a function or to execute a software module. The software/firmware stored in the ROM 214 then causes the microprocessor 212 to issue a command to a motor attached to the drive wheel 201 through the motor interface 217 to rotate the shaft 204 in a clockwise direction. The software/firmware controls the extent of the rotation. At step S4, the amount of rotation is sufficient to lance the user's digit with the lancet 309. The software/firmware stored in the ROM 214 then causes the microprocessor 212 to control the motor to rotate the shaft 204 in the opposite direction, at step S5. As the test disc member rotates anticlockwise, milking occurs at step S6.

Firstly, at step S6A, there is no pressure applied by the test disc member on the digit. At step S6B, there is a medium amount of pressure on the digit. At step S6C, there is low or no pressure applied by the test disc member on the digit. At this point, the digit coincides with the part of the test disc member that is immediately before the blood collection part 315.

At step S7, the software/firmware causes the microprocessor 212 to control the motor to cease rotation when the shaft 214 is such that the blood collection part 315 is coincident with the aperture 105, and thus the user's digit. At this point, the analyte interface circuit 219 is coupled directly to the blood analyte measuring part 316, which by action of the blood collection part 315 has been provided with blood from the user's digit. At step S8, analyte measurement is performed. This involves the analyte interface circuit 219 providing voltages to the electrical connection contacts 318, and thus to the blood analyte measuring part 316, and measuring parameters of resulting signals. The measured parameters, particularly voltage parameters, are used by the software/firmware stored in the ROM 214, as executed by the processor 212, to calculate a blood glucose measurement level of the user. The blood glucose measurement is then caused by the software/firmware to be displayed on the display 104 through action of the microprocessor 212 on the display drive 216.

At step S9, the software/firmware results in the microprocessor 212 controlling the drive disc 201 to rotate anticlockwise. Rotation continues until the notch 301 on the test disc member is coincident with the guide 206. At step S10, the test disc member rises up the cartridge 106. In the case where biasing of the test discs up the cartridge 106 is provided by a bias means, for instance a spring, step S10 requires no action on part of the software/firmware and microprocessor 212, although there may be a pause before the next step. In embodiments where movement of the test disc members along the shaft 204 occurs through driving action, step S10 involves the microprocessor 212, under control of the software/firmware stored in the ROM 214, controlling a motor through the motor interface 217. Subsequently, at step S11, the microprocessor 212, under control of the software/firmware stored in the ROM 214, causes the shaft 204 to rotate again in a clockwise direction and to cease rotating when the drive dog 320 engages with the drive slot 307 of the next test disc member in the cartridge 106. At this stage, the test disc members rise up the cartridge 106 slightly.

The operation ends at step S12.

Instead of the blood collection part 315 being located next to the third position 314, i.e. bounding only the part of the disc edge 305 that is purely circumferential, the blood collection part could instead be located on the disc edge 305 at the junction between the cutaway portion 302 and the circumferential portion. The blood collection part 315 in this instance may extend for between 0.5 mm and 2 mm along the disc edge 305 at the cutaway portion 302. The blood collection part 315 in this instance may also extend for between 0.5 mm and 2 mm along the disc edge 305 at the circumferential part.

Alternatively or additionally, the analyte measuring part 316 may be sandwiched between two layers of wicking material, the wicking material causing the blood to be drawn through the analyte measuring part 316.

Although in the above the shaft 204 is said to be driven by a drive wheel 201 that is coupled to the shaft 204 by a drive belt 202, the drive may instead be direct (i.e. the drive mechanism is coupled directly to the shaft 204), or connection may be made by a notched belt, a vee belt, or by a direct gear mechanism. Instead of an electric motor, a clockwork drive could be used. A clockwork drive mechanism has a number of advantages, particularly where access to batteries or battery chargers or electricity supplies are limited. In the embodiments in which a clockwork mechanism is used, the user can be sure that the BGM 100 will not cease operating because of drained batteries. A clockwork mechanism may be particularly suited to developing countries and emerging markets.

In embodiments in which an electrical motor is used to drive the shaft 204, preferably control is exerted over the motor by software. In this way, the speed of rotation can easily be controlled. Additionally, the extent of rotation can more easily be controlled. The motor may be a stepper motor.

Alternatively, a mechanical drive arrangement may be present, for instance using a lever or other device for manual actuation. A suitable mechanism may be one similar to those previously used in SLR cameras.

The conductive tracks 317 and the contact pads 318 may be formed by leadframe. Alternatively, overmoulding may be used. Alternatively, printed circuit board (PCB) printing may be used.

Optionally, each of the test disc members 209, 210, 600 is separated from adjacent test disc members by a membrane (not shown in the drawings). In this case, the membrane preferably fits closely to the internal surface of the hollow cylindrical housing part 203. An effect of the membrane is to reduce the possibility of disc cross-contamination. Use of a membrane may allow the test disc members 208 to 210, 600 to have a reduced separation than would be the case without the use of a membrane.

In the above, the test disc members 208 to 210, 600 are said to be biased upwards by a bias means, for instance a compression spring. Alternative mechanisms for moving the test disc members 208 to 210, 600 up the cartridge may be used. For instance, a threaded lifting cam may be provided on the shaft 204 or alternatively on the interior surface of the hollow cylindrical housing part 203. Alternatively, the test disc members 208 to 210, 600 may remain stationary, with the aperture 105 and the drive dog 320 instead being moved along the axis of the cartridge 106. Movement of the aperture 105 may be achieved by the use of a sliding door in an elongated slot. Movement of the door allows a different strip to be revealed at the aperture 105.

Instead of the blood collection part 315 wicking blood towards the analyte measuring part 316, blood may be communicated to the analyte measuring part 316 instead through gravity.

Additionally, the test disc members 208 to 210, 600 may include a disinfecting or cleaning portion that contacts the digit before lancing. This can reduce risk of infection of the wound and also can increase accuracy in particular by removing any glucose from the skin (as may occur after eating fruit etc.).

Additionally or alternatively, the test disc members 208 to 210, 600 may include a cleaning portion that is arranged to contact the digit subsequent to the blood collection part 305. This can remove additional blood from the finger, and may also serve to assist closure of the puncture.

## Claims

1. A cartridge configured to be inserted into a meter, the cartridge comprising:
a housing having at least two electrical contacts disposed on an inner surface of a wall of the housing and at least two electrical contacts disposed on an outer surface of the wall of the housing, wherein a conductive path is provided between corresponding ones of the at least two electrical contacts on the inner and outer surfaces; and
at least one testing member supported within the housing, each testing member having at least two contact pads configured to engage with the at least two electrical contacts disposed on the inner surface of the housing wall when that testing member is moved into a measurement position, wherein the at least two electrical contacts disposed on the outer surface of the housing wall are configured to provide a conductive connection between the testing member and the meter.

2. A cartridge according to claim 1, wherein the at least two contact pads of each testing member are disposed on an edge of each testing member.

3. A cartridge according to claim 1 or claim 2, wherein the wall of the housing is a side wall.

4. A cartridge according to any preceding claim, wherein the housing has three electrical contacts disposed on an inner surface of the wall of the housing and three electrical contacts disposed on an outer surface of the wall of the housing.

5. A cartridge according to any preceding claim, wherein the contacts disposed on the inner surface are brush contacts.

6. A cartridge according to any of claims 1 to 4, wherein the contacts disposed on the inner surface are spring contacts.

7. Apparatus comprising a cartridge according to any preceding claim and a meter configured to retain the cartridge, the meter configured:
to detect whether the cartridge is correctly inserted into the meter; and
to permit movement of the testing members only when it is determined that the cartridge is correctly inserted.

8. Apparatus according to claim 7, wherein the meter further comprises at least two contacts configured to engage with the at least two electrical contacts disposed on the outer surface of the wall of the housing when the cartridge is correctly inserted so as to provide the conductive connection between the testing member and the meter.

9. Apparatus according to claim 7 or 8, wherein the meter further comprises a proximity sensor configured to enable the meter to detect the presence of the cartridge within the meter.

10. Apparatus according to any of claims 7 to 9, wherein the meter further comprises a mechanical switch configured to be engaged by the housing of the cartridge such as to enable the meter to detect the presence of the cartridge within the meter.

11. A cartridge or apparatus according to any preceding claim, wherein each testing member supports a lancet which protrudes form an edge of each testing member.

## Patentansprüche

1. Kartusche, die so ausgelegt ist, dass sie in ein Messgerät eingeführt wird, wobei die Kartusche aufweist:
ein Gehäuse mit mindestens zwei elektrischen Kontakten, die auf einer Innenfläche einer Wand des Gehäuses angeordnet sind, und mindestens zwei elektrischen Kontakten, die auf einer Außenfläche der Wand des Gehäuses angeordnet sind, wobei eine Leiterbahn zwischen entsprechenden der mindestens zwei elektrischen Kontakte auf den Innen- und Außenflächen vorgesehen ist;
mindestens ein Prüfelement, das innerhalb des Gehäuses gehalten wird, wobei jedes Prüfelement mindestens zwei Kontaktstellen aufweist, die so ausgelegt sind, dass sie in die mindestens zwei elektrischen Kontakte eingreifen, die auf der Innenfläche der Gehäusewand angeordnet sind, wenn dieses Prüfelement in eine Messposition bewegt wird, wobei die mindestens zwei elektrischen Kontakte, die auf der Außenfläche der Gehäusewand angeordnet sind, so ausgelegt sind, dass sie eine leitende Verbindung zwischen dem Prüfelement und dem Messgerät bereitstellen.

2. Kartusche nach Anspruch 1, wobei die mindestens zwei Kontaktstellen jedes Prüfelements auf einer Kante eines jeden Prüfelements angeordnet sind.

3. Kartusche nach Anspruch 1 oder 2, wobei die Wand des Gehäuses eine Seitenwand ist.

4. Kartusche nach einem der vorhergehenden Ansprüche, wobei das Gehäuse drei elektrische Kontakte, die auf einer Innenfläche der Wand des Gehäuses angeordnet sind, und drei elektrische Kontakte aufweist, die auf einer Außenfläche der Wand des Gehäuses angeordnet sind.

5. Kartusche nach einem der vorhergehenden Ansprüche, wobei die Kontakte, die auf der Innenfläche angeordnet sind, Bürstenkontakte sind.

6. Kartusche nach einem der Ansprüche 1 bis 4, wobei die Kontakte, die auf der Innenfläche angeordnet sind, Federkontakte sind.

7. Vorrichtung, umfassend eine Kartusche nach einem der vorhergehenden Ansprüche und Messgerät, das so ausgelegt ist, dass es die Kartusche aufnimmt, wobei das Messgerät ausgelegt ist zum:
Erkennen, ob die Kartusche korrekt in das Messgerät eingeführt ist; und
Zulassen von Bewegung der Prüfelemente nur dann, wenn bestimmt wird, dass die Kartusche korrekt eingeführt ist.

8. Vorrichtung nach Anspruch 7, wobei das Messgerät ferner mindestens zwei Kontakte aufweist, die so ausgelegt sind, dass sie in die mindestens zwei elektrischen Kontakte eingreifen, die auf der Außenfläche der Wand des Gehäuses angeordnet sind, wenn die Kartusche korrekt eingeführt ist, um die leitende Verbindung zwischen dem Prüfelement und dem Messgerät bereitzustellen.

9. Vorrichtung nach Anspruch 7 oder 8, wobei das Messgerät ferner einen Näherungssensor aufweist, der so ausgelegt ist, dass er das Messgerät zum Erkennen des Vorhandenseins der Kartusche innerhalb des Messgeräts befähigt.

10. Vorrichtung nach einem der Ansprüche 7 bis 9, wobei das Messgerät ferner einen mechanischen Schalter aufweist, der so ausgelegt ist, dass er durch das Gehäuse der Kartusche in Eingriff gebracht wird, um das Messgerät zum Erkennen des Vorhandenseins der Kartusche innerhalb des Messgeräts zu befähigen.

11. Kartusche oder Vorrichtung nach einem der vorhergehenden Ansprüche, wobei jedes Prüfelement eine Lanzette trägt, die von einer Kante eines jedes Prüfelements vorsteht.

## Revendications

1. Cartouche configurée de façon à être insérée dans un instrument de mesure, cette cartouche comprenant :
un boîtier ayant au moins deux contacts électriques disposés sur une surface interne d'une paroi du boîtier et au moins deux contacts électriques disposés sur une surface externe de la paroi du boîtier, dans lequel un chemin conducteur est prévu entre les contacts correspondants des au moins deux contacts électriques sur les surfaces interne et externe ; et
au moins un élément d'analyse supporté à l'intérieur du boîtier, chaque élément d'analyse ayant au moins deux plots de contact configurés de façon à s'engager avec les au moins deux contacts électriques disposés sur la surface interne de la paroi du boîtier lorsque cet élément d'analyse est mis dans une position de mesure, les au moins deux contacts électriques disposés sur la surface externe du boîtier étant configurés de façon à fournir une connexion conductrice entre l'élément d'analyse et l'appareil de mesure.

2. Cartouche selon la revendication 1, dans laquelle les au moins deux plots de contact de chaque élément d'analyse sont disposés sur un bord de chaque élément d'analyse.

3. Cartouche selon la revendication 1 ou la revendication 2, dans laquelle la paroi du boîtier est une paroi latérale.

4. Cartouche selon l'une quelconque des revendications précédentes, dans laquelle le boîtier a trois contacts électriques disposés sur une surface interne de la paroi du boîtier et trois contacts électriques disposés sur une surface externe de la paroi du boîtier.

5. Cartouche selon l'une quelconque des revendications précédentes, dans laquelle les contacts disposés sur la surface interne sont des contacts à balai.

6. Cartouche selon l'une quelconque des revendications 1 à 4, dans laquelle les contacts disposés sur la surface interne sont des contacts à ressort.

7. Appareil comprenant une cartouche selon l'une quelconque des revendications précédentes et un appareil de mesure configuré de façon à retenir la cartouche, cet appareil de mesure étant configuré de façon à :
détecter si la cartouche est insérée correctement dans l'appareil de mesure ; et à
ne permettre le mouvement des éléments d'analyse que lorsqu'il est déterminé que la cartouche est insérée correctement.

8. Appareil selon la revendication 7, dans lequel l'appareil de mesure comprend au moins deux contacts configurés de façon à s'engager avec les au moins deux contacts électriques disposés sur la surface externe de la paroi du boîtier lorsque la cartouche est insérée correctement de façon à fournir la connexion conductrice entre l'élément d'analyse et l'appareil de mesure.

9. Appareil selon la revendication 7 ou 8, dans lequel l'appareil de mesure comprend en outre un capteur de proximité configuré de façon à permettre à l'appareil de mesure de détecter la présence de la cartouche à l'intérieur de l'appareil de mesure.

10. Appareil selon l'une quelconque des revendications 7 ou 9, dans lequel l'appareil de mesure comprend en outre un commutateur mécanique configuré de façon à être engagé par le boîtier de la cartouche de façon à permettre à l'appareil de mesure de détecter la présence de la cartouche à l'intérieur de l'appareil de mesure.

11. Cartouche ou appareil selon l'une quelconque des revendications précédentes, dans lesquels chaque élément d'analyse supporte une lancette qui fait saillie depuis un bord de chaque élément d'analyse.
